Europäisches Patentamt

European Patent Office

Office européen des brevets

(19)

(11) Publication number: **0 072 995**
**B1**

(12) **EUROPEAN PATENT SPECIFICATION**

(45) Date of publication of patent specification: **25.06.86**

(21) Application number: **82107437.4**

(22) Date of filing: **16.08.82**

(51) Int. Cl.⁴: **C 01 F 7/00,** C 03 C 1/00,
C 03 C 3/00

(54) **Clear aluminum oxide solutions and glasses.**

(30) Priority: **17.08.81 US 293820**

(43) Date of publication of application:
**02.03.83 Bulletin 83/09**

(45) Publication of the grant of the patent:
**25.06.86 Bulletin 86/26**

(84) Designated Contracting States:
**BE DE FR GB IT NL**

(56) References cited:
**FR-A-1 524 490**
**US-A-3 056 725**
**US-A-3 394 990**
**US-A-3 941 719**
**US-A-4 176 171**
**US-A-4 278 632**

(73) Proprietor: **WESTINGHOUSE ELECTRIC
CORPORATION
Westinghouse Building Gateway Center
Pittsburgh Pennsylvania 15222 (US)**

(72) Inventor: **Yoldas, Bulent Erturk
1605 Jamestown Place
Pittsburgh Pennsylvania (US)**

(74) Representative: **Fleuchaus, Leo, Dipl.-Ing. et al
Fleuchaus & Wehser Melchiorstrasse 42
D-8000 München 71 (DE)**

Courier Press, Leamington Spa, England.

## Description

A process has recently been discovered for preparing polymerized metal oxide glasses of titanium or tantalum from solutions of their alkoxides. This process has the advantage that the coatings can be prepared at relatively low temperatures from solutions of carefully controlled composition. The resulting coatings can be made to have a particular index of refraction by controlling the composition.

However, until now the compositions were limited to those containing principally titanium and tantalum alkoxides. It was not possible, for example, to prepare a polymeric alumina glass from an aluminum alkoxide, although alumina powders could be prepared from an aluminum alkoxide solution.

An article by B. E. Yoldas in the Journal of Material Science, Volume 12, 1977, pps. 1203 to 1208, titled "Preparation of Glasses and Ceramics from Metal-Organic Compounds", discloses the hydrolysis of aluminum secondary butoxide to form aluminum oxide which then decomposes under heat to form alumina.

U.S. Patent 3,357,791 discloses a process for preparing colloidal size particles of alpha alumina monohydrate from aluminum alkoxides.

An article by Bulent E. Yoldas in the Journal of Applied Chemistry and Biotechnology, Volume 23, 1973, pps. 803 to 809, titled "Hydrolysis of Aluminum Alkoxides and Berrite Conversion", discloses the hydrolysis of aluminum alkoxides to form aluminum hydroxides which are then polymerized to form alumina.

U.S. Patents 3,944,658 and 3,941,719 disclose the preparation of a non-particulate alumina from aluminum alkoxides in the presence of an acid.

U.S. Patent 3,056,725 relates to the production of polymeric aluminum oxide hydroxides of hydroxy aluminum oxide polymers, and to such compounds as new compositions of matter. The processes involve the hydrolysis of aluminum alcoholates with water in the presence of alcohols under unique procedures and conditions which form hydroxides, instead of hydrates, of aluminum.

The principal object of the invention is to provide an improved clear solution.

The invention resides broadly in a method of preparing a clear, partially hydrolyzed aluminum alkoxide solution by mixing $Al(OR)_3$, wherein R is an alkyl to $C_6$, with 0.4 to 1 mole of water per mole of $Al(OR)_3$ and alcohol and heating the mixture, characterized in that said mixing is performed in the presence of sufficient alcohol to give a maximum weight % of equivalent $Al_2O_3$ of 10%, and heating said mixture until clear.

It has been discovered how to make a clear solution of an aluminum alkoxide from which a polymerized alumina glass can be formed. While the process of this invention requires that certain parameters be carefully controlled, the process itself is not difficult to perform and can be carried out with relatively inexpensive equipment. Unlike prior alumina solutions which were prepared from hydroxides of alkoxides in water and peptizing the resulting precipitate by using an acid, the alumina solutions of this invention are prepared in alcohol in the absence of acid. Also, they are not cloudy or translucent like the former solutions, but can be made perfectly clear, indicating the absence of light-scattering particles. The polymerized alumina glasses of this invention can be prepared from these solutions in bulk or as coatings.

In the process of this invention a composition is first prepared of an aluminum alkoxide, an alcohol, and water. The aluminum alkoxides suitable for use in this invention have the general formula $Al(OR)_3$, where R is alkyl to $C_6$. Preferably, R is secondary butyl because aluminum secondary butoxide is a liquid. and is inexpensive.

The alcohol used in the composition should be water-free and a solvent for the aluminum alkoxide. Also, the alcohol should preferably be the same alcohol that is formed when the aluminum alkoxide is hydrolyzed, so that it is not necessary to separate two different alcohols. That is, the alcohol would preferably have the formula ROH where R is the same R that is present in the aluminum alkoxide. Higher boiling alcohols are preferred as higher temperatures can then be used to form the composition without boiling off the alcohol. However, generally, the alcohol is chosen on the basis of cost and the properties of the liquid desired for a particular application.

In forming the composition the aluminum alkoxide and the water should only be mixed in the presence of the alcohol. That is, the alcohol may be mixed with the alkoxide first followed by the addition of the water or, in the preferred procedure, the alcohol and the water are mixed together first and then the alkoxide is added. The addition of the water directly to the alkoxide should be avoided as it can result in an inhomogeneous hydrolyzation of the alkoxide.

The amount of water used in the composition is highly critical as if less than about 0.4 or more than about 1 mole of water is used per mole of alkoxide the liquid will contain precipitate and will not form a clear solution. The maximum amount of aluminum alkoxide in the initial solution, calculated as equivalent $Al_2O_3$ by weight, should not exceed 12 percent as a higher concentration will result in an incomplete dissolution of precipitate and an unclear solution. It is not necessary for any acid to be present in the composition and preferably no acid should be used.

Once the alkoxide, alcohol, and water have been mixed a precipitate will form which must be redissolved. This can be accomplished by heating the composition until it clears. The composition is preferably heated to at least 40°C as lower temperatures require too much time for the composition to clear. The composition is preferably not heated to temperatures greater than 60°C, however, as at higher

# 0 072 995

temperatures alcohol is evaporated, although higher temperatures will still dissolve the precipitate as long as the water concentration is between about 0.4 to about 1.0 moles/mole alkoxide.

Once the liquid is clear it may be necessary to add additional water in order to complete the hydrolysis. The additional water introduced should preferably be sufficient to raise the moles of water to moles of alkoxide ratio to about 2 to about 3 as at a ratio of less than about 2 the solution will contain a large number of unhydrolyzed alkyl bonds which result in the deposition of carbon during the pyrolysis. At a ratio of greater than about 3 the composition tends to gel at low temperatures which makes coating difficult, although the resultant alumina is clear. The additional water that is added should preferably be in alcohol to avoid contacting the solution with high concentrations of water. Sufficient alcohol is preferably mixed with the additional water so that the aluminum alkoxide in the composition, calculated as equivalent alumina, is not more than 3 percent as higher concentrations may produce a gel. This alumina solution may be combined with other alkoxides or various other solutions to obtain a wide variety of glass or ceramic compositions.

The completed solution is stable and can be stored until it is ready to be used. To use the solutions to prepare alumina coatings the solution can be sprayed, dipped, or otherwise applied to the surface of any substrate which can withstand the curing temperature. The alumina coatings of this invention are useful for protecting surfaces, for passivation of surfaces, as a desiccant, as antireflective coatings on surfaces, as optical coatings, or for other purposes. Bulk glass or ceramic objects can also be prepared from the solution by gelling it alone or with additions, then drying and curing.

In order to remove water, alcohol, and residual organics which are still present in the composition, the composition must be heated at at least 400 to 500°C until these compounds are no longer emanate from it and a pure oxide is attained. The cured oxide is totally transparent and, unlike prior aluminum oxide prepared from aqueous alkoxide derived solutions is not cloudy.

The following example further illustrates this invention.

## Example 1

Samples were made by adding various amounts of water to 140 g of dry ethyl alcohol, then adding 24.8 g (0.1 m) of $Al(OC_4H_9)_3$ into these liquids. The samples were kept at 50°C for 2 hours. The following table gives the results.

| Sample # | $H_2O$ (mol/moles alk) | Condition of sample after 2 hrs. at 50°C |
|---|---|---|
| 1 | 0.15 (.27 g) | Undissolved precipitate |
| 2 | 0.30 (.54 g) | Undissolved precipitate |
| 3 | 0.40 (.72 g) | Cleared, slight precipitate |
| 4 | 0.52 (.90 g) | Totally clear |
| 5 | 0.70 (1.26 g) | Totally clear |
| 6 | 1.00 (1.80 g) | Totally clear |
| 7 | 1.20 (2.16 g) | Some precipitate remaining at bottom |
| 8 | 1.50 (2.70 g) | Milky |
| 9 | 2.00 (3.60 g) | Milky |

The table shows that the only samples which cleared were those which had between 0.4 and 1.0 moles of water per mole of alkoxide.

## Example 2

In these experiments, 2.34 g (1.3 m/m) $H_2O$ was mixed with 140 g alcohol and this mixture was added to sample #5 from Example 1, which contained about 3% equivalent $Al_2O_3$. The solution remained clear. It now had a water content of 2 moles/per mole of alkoxide, and an alkoxide content of 1.5% equivalent $Al_2O_3$.

A glass slide was dipped into the solution and was heated to 600°C to form a clear polymerized alumina coating.

## Example 3

Example 1 was repeated using 1 g of water and 25 g of aluminum secondary butoxide to show the effect of altering the concentration of alkoxide in the solution. The following table gives the results.

3

| Sample | Ethyl alcohol | % e.g. $Al_2O_3$ | Condition after 2 hrs. at 50°C |
|---|---|---|---|
| 1 | 100 g | 4% | Clear |
| 2 | 58 g | 6 | Clear |
| 3 | 37 g | 8 | Clear |
| 4 | 24 g | 10 | Clear |
| 5 | 15 g | 12 | Some precipitate |
| 6 | 7 g | 14 | Cloudy |

The above table shows that the maximum equivalent $Al_2O_3$ concentration in the initial solution should be 10% or less.

Example 4

Sample 1 in Example 3 was prepared using isopropyl and sec-butyl alcohol instead of ethyl alcohol. Clear solutions resulted. When a clear solution is produced a clear oxide coating can always be produced by adding the additional water and heating to at least 500°C. However, if the solution is to be used, along with other oxide constituents, as a source of alumina, then the solution can be used without introducing additional water before mixing with other components.

**Claims**

1. A method of preparing a clear, partially hydrolyzed aluminum alkoxide solution by mixing $Al(OR)_3$, wherein R is an alkyl to $C_6$, with 0.4 to 1 mole of water per mole of $Al(OR)_3$ and alcohol and heating the mixture, characterized in that said mixing is performed in the presence of sufficient alcohol to give a maximum weight % of equivalent $Al_2O_3$ of 10%, and heating said mixture until clear.

2. A method according to claim 1 including the additional subsequent step of adding sufficient additional water to bring the number of moles of water per mole of alkoxide up to at least about 2, in the presence of sufficient alcohol to give a maximum weight % of equivalent $Al_2O_3$ of about 3%.

3. A method according to claim 1 or 2 wherein R is secondary butyl.

4. A method according to claim 1, 2 or 3 wherein said mixture is heated at about 40 to about 60°C.

5. A method according to any of the preceding claims when appended to claim 2 wherein the number of moles of water per mole of alkoxide is about 2 to about 3.

6. A method according to any of the preceding claims wherein said water is mixed with said alcohol and that mixture is mixed with said alkoxide.

7. A method according to any of the preceding claims wherein said alcohol and said alkoxide are mixed prior to the addition of said water.

8. A method according to any of the preceding claims wherein said alcohol is ROH and the R group in said alcohol is the same as the R group in said alkoxide.

9. A method according to any one of claims 1 to 7 wherein said alcohol is selected from the group consisting of ethanol, propanol, butanol, and mixtures thereof.

10. The clear solution prepared according to the method of any of the preceding claims.

11. A method using the solution according to claim 10 wherein the mixture is heated to a temperature of at least about 400°C.

12. A method using the solution according to claim 10 or 11 wherein a substrate is coated with said mixture and heat treated.

**Patentansprüche**

1. Verfahren zur Herstellung einer klaren, teilweise hydrolysierten Aluminium-Alkoxid-Lösung durch Mischen von $Al(OR)_3$, worin OR ein Alkyl zu $C_6$ ist, mit 0,4 bis 1 Mol Wasser pro Mol $Al(OR)_3$ und Alkohol und Erhitzen der Mischung, dadurch gekennzeichnet, daß das genannte Mischen in Anwesenheit von ausreichend Alkohol erfolgt, um einen maximalen Gewichtsprozentsatz von äquivalenten $Al_2O_3$ von 10% zu ergeben; und daß die genannte Mischung erhitzt wird, bis sie klar ist.

2. Verfahren nach Anspruch 1, in dem der zusätzliche nachfolgende Schritt enthalten ist, daß ausreichend zusätzliches Wasser beigegeben wird, um die Anzahl Mole Wasser pro Mol Alkoxid auf mindestens ungefähr zwei zu bringen, unter Anwesenheit von ausreichend Alkohol, um einen maximalen Gewichtsprozentsatz von äquivalentem $Al_2O_3$ von ungefähr 3% zu ergeben.

3. Verfahren nach Anspruch 1 oder 2, bei dem OR sekundäres Butyl ist.

**0 072 995**

4. Verfahren nach einem der Ansprüche 1, 2 oder 3, bei dem die genannte Mischung bei ungefähr 40° bis ungefähr 60° erhitzt wird.

5. Verfahren nach einem der vorstehenden Ansprüche, wenn sie an Anspruch 2 angefügt werden, bei dem die Anzahl der Mole Wasser pro Mol Alkoxid ungefähr 2 bis 3 ist.

6. Verfahren nach einem der vorstehenden Ansprüche, bei dem das genannte Wasser mit dem genannten Alkohol und diese Mischung mit dem genannten Alkoxid gemischt wird.

7. Verfahren nach einem der vorstehenden Ansprüche, bei dem der genannte Alkohol und das genannte Alkoxid vor der Zufügung des genannten Wassers gemischt werden.

8. Verfahren nach einem der vorstehenden Ansprüche, bei dem der genannte Alkohol ROH ist und die R-Gruppe im genannten Alkohol die gleiche ist wie die R-Gruppe im genannten Alkoxid.

9. Verfahren nach einem der Ansprüche 1 bis 7, bei dem der genannte Alkohol aus der aus Äthanol, Propanol, Butanol und deren Mischungen bestehenden Gruppe ausgewählt wird.

10. Klare Lösung, die gemäß dem Verfahren nach einem der vorstehenden Ansprüche dargestellt wurde.

11. Verfahren zur Verwendung der Lösung nach Anspruch 10, bei dem die Mischung auf eine Temperatur von mindestens 400°C erhitzt wird.

12. Verfahren zur Verwendung der Lösung nach Anspruch 10 oder 11, bei dem ein Substrat mit der genannten Mischung beschichtet wird und anschließend eine Wärmebehandlung erfährt.

## Revendications

1. Procédé de préparation d'une solution limpide d'alcoxyde d'aluminium partiellement hydrolysée par mélange d'Al(OR)$_3$, R étant un radical alkyle ayant jusqu'à 6 atomes de carbone, avec 0,4 à 1 mole d'eau par mole d'Al(OR)$_3$ et de l'alcool, et par chauffage du mélange, caractérisé par le fait qu'on effectue ledit mélange en présence de suffisamment d'alcool pour obtenir un pourcentage en poids maximum d'Al$_2$O$_3$ équivalente égal à 10%, et par chauffage dudit mélange jusqu'à ce qu'il soit limpide (ou: à clarté dudit mélange).

2. Procédé selon la revendication 1 comprenant l'étape subséquente supplémentaire d'addition de suffisamment d'eau supplémentaire pour porter le nombre de moles d'eau par mole d'alcoxyde à au moins 2 environ, en présence de suffisamment d'alcool pour obtenir un pourcentage en poids maximum d'environ 3% eu Al$_2$O$_3$ équivalente.

3. Procédé selon la revendication 1 ou 2, dans lequel R est un radical butyle secondaire.

4. Procédé selon les revendications 1, 2 ou 3, dans lequel ledit mélange est chauffé à une température d'environ 40 à environ 60°C.

5. Procédé selon l'une quelconque des revendications précédentes lorsque rattaché à la revendication 2, dans lequel le nombre de moles d'eau par mole d'alcoxyde va d'environ 2 à environ 3.

6. Procédé selon l'une quelconque des revendications précédentes, dans lequel l'eau est mélangée avec l'alcool et ce mélange est mélangé avec l'alcoxyde.

7. Procédé selon l'une quelconque des revendications précédentes, dans lequel l'alcool et l'alcoxyde sont mélangés avant l'addition de l'eau.

8. Procédé selon l'une quelconque des revendications précédentes, dans lequel l'alcool est ROH et le groupe R dans cet alcool est identique au groupe R dans l'alcoxyde.

9. Procédé selon l'une quelconque des revendications 1 à 7, dans lequel l'alcool est choisi parmi les éthanol, propanol, butanol et leurs mélanges.

10. Solution limpide préparée selon le procédé de l'une quelconque des revendications précédentes.

11. Procédé utilisant la solution selon la revendication 10, dans lequel on chauffe le mélange à une température d'au moins 400°C environ.

12. Procédé utilisant la solution selon la revendication 10 ou 11, dans lequel un support est revêtu avec le mélange et subit un traitement thermique.